# FASCICULE DE BREVET EUROPEEN

(11) **EP 4 069 182 B1**
(45) Date de publication et mention de la délivrance du brevet: **25.03.2026**
(21) Numéro de dépôt: 20824319.6
(22) Date de dépôt: 26.11.2020
(51) Int. Cl.: A61K 8/03, A61K 8/31, A61K 8/34, A61K 8/92, A61Q 1/14

(54) **COMPOSITION COSMÉTIQUE TRIPHASIQUE**
DREIPHASIGE KOSMETISCHE ZUSAMMENSETZUNG
THREE-PHASE COSMETIC COMPOSITION

(30) Priorité: 03.12.2019 FR 1913665
(43) Date de publication de la demande: 12.10.2022
(73) Titulaire: Laboratoires M & L, 04100 Manosque (FR)
(72) Inventeur: PAPPATICO, Sabrina, 13100 AIX EN PROVENCE (FR); VERRECCHIA, Nicolas, 69003 LYON (FR); LARTAUD, Alice, 04100 MANOSQUE (FR)
(74) Mandataire: Renard, Emmanuelle
(86) Numéro de dépôt international: PCT/FR2020/052200
(87) Numéro de publication internationale: WO 2021/111065

(56) Documents cités:
- EP-A2- 1 327 439
- WO-A1-2014/018543
- WO-A2-2014/082794
- FR-A1- 2 655 265
- FR-A1- 2 939 662
- GB-A- 2 259 015

## Description

### OBJET DE L'INVENTION

La présente invention concerne une composition cosmétique triphasique comprenant une phase inférieure aqueuse, une phase intermédiaire huileuse non siliconée et une phase supérieure huileuse non siliconée. Elle concerne également l'utilisation cosmétique de cette composition pour le soin, le nettoyage et/ou le parfumage de la peau et/ou des cheveux et/ou le démaquillage de la peau, notamment du visage, ainsi qu'un procédé cosmétique utilisant cette composition.

### ARRIERE-PLAN DE L'INVENTION

Il existe sur le marché des compositions cosmétiques constituées de trois phases visuellement distinctes. Ces compositions triphasiques se distinguent des émulsions triples qui sont, au repos, constituées d'une dispersion, généralement non visible à l'oeil nu, de gouttelettes d'émulsion huile-dans-eau dispersées dans une phase huileuse continue ou de gouttelettes d'émulsion eau-dans-huile dans une phase aqueuse continue. Dans les compositions triphasiques, la phase inférieure au repos est généralement constituée de la phase aqueuse, qui est la plus dense, l'une au moins des phases intermédiaire et supérieure étant une phase huileuse. Chacune de ces phases est séparée de la phase adjacente par une seule interface continue, qui est généralement horizontale. Cette interface peut être mise en évidence par l'utilisation de colorants distincts dans chacune des phases. Les compositions triphasiques nécessitent d'être agitées avant emploi pour former une émulsion extemporanée. Cette dernière doit être suffisamment stable pour permettre une application simultanée des trois phases, mais telle qu'au repos, les trois phases se séparent rapidement pour retrouver leur état initial. Un exemple de composition triphasique renfermant une phase inférieure aqueuse, une phase aqueuse intermédiaire et une phase supérieure huileuse est décrit dans le document WO 2019/002579. D'autres systèmes de compositions cosmétiques triphasiques sont connues également (voir notamment les demandes de brevet FR 2 655 265 A1, WO 2014/018543 A1, GB 2 259 015 A et US 2015/258006 A1).

De nombreuses compositions triphasiques sont par ailleurs disponibles dans le commerce et sont en particulier utilisées comme lotions de démaquillage de la peau ou comme brumes de soin et/ou de protection des cheveux. La plupart comprennent toutefois une proportion importante d'huile de silicone (dimethicone) ou de paraffine (huile minérale). Or, les consommateurs cherchent de plus en plus à disposer de produits cosmétiques renfermant des ingrédients d'origine naturelle et se détournent ainsi des produits renfermant des huiles de synthèse ou d'origine pétrochimique, telles que les silicones et les huiles minérales.

Quelques compositions ont ainsi été proposées, qui sont dépourvues des composés précités (notamment le Sérum régénérateur anti-chute triphasique de René Furterer). Ces compositions renferment toutefois une proportion importante d'alcool qui les rend inadaptées à une utilisation pour le démaquillage des yeux ou le démaquillage complet du visage.

Une autre solution proposée par la société SEPPIC consiste à utiliser une combinaison d'huile de ricin et d'Emogreen^{®} L15, qui est un mélange d'alcanes linéaires en C₁₅-C₁₉ d'origine végétale. Cette combinaison permet de formuler une composition triphasique constituée d'une phase aqueuse inférieure, d'une phase intermédiaire huileuse polaire, à base d'huile de ricin, et d'une phase supérieure huileuse apolaire, à savoir l'Emogreen^{®} L15. L'obtention d'une interface nette entre la phase aqueuse et la phase huileuse intermédiaire nécessite l'ajout d'un tensioactif particulier à base d'alkylpolyglycoside dans cette dernière et d'un sel dans la première, ce qui restreint la latitude du formulateur. Surtout, les proportions recommandées pour obtenir une composition d'aspect satisfaisant correspondent à un ratio phases grasses / phase aqueuse de 70/30. La composition obtenue a par conséquent un toucher gras qui convient pour l'application capillaire recommandée mais pas à une utilisation comme lotion démaquillante. La Demanderesse a en outre démontré qu'en augmentant la proportion de phase aqueuse, les interfaces obtenues n'étaient pas nettes lorsque la composition était remise au repos après agitation.

A la connaissance de la Demanderesse, il n'a donc jamais été proposé de composition triphasique dépourvue d'huile de silicone, renfermant au moins 50% en poids de phase aqueuse et deux phases huileuses distinctes, et qui présente des interfaces nettes au repos.

Après de nombreuses recherches, la Demanderesse est parvenue à concilier toutes ces exigences grâce à un choix judicieux des constituants des trois phases.

### RESUME DE L'INVENTION

L'invention a ainsi pour objet une composition cosmétique triphasique comprenant : (a) une phase inférieure aqueuse représentant au moins 50% du poids de la composition et renfermant de 2 à 15% en poids de glycérine, par rapport au poids total de la composition, (b) une phase intermédiaire huileuse non siliconée renfermant de l'huile de ricin et éventuellement au moins une autre huile végétale, et (c) une phase supérieure huileuse non siliconée renfermant au moins un alcane linéaire ou ramifié en C₁₀-C₂₀, étant entendu que, lorsque la phase intermédiaire huileuse renferme au moins une autre huile végétale, le rapport en poids de l'huile de ricin à l'huile végétale est d'au moins 65:35

Elle a également pour objet l'utilisation cosmétique de cette composition pour le soin, le nettoyage et/ou le parfumage de la peau et/ou des cheveux et/ou le démaquillage de la peau, notamment du visage.

Elle a encore pour objet un procédé cosmétique de soin, de nettoyage et/ou et/ou de parfumage de la peau et/ou des cheveux et/ou de démaquillage de la peau, notamment du visage, comprenant l'application topique sur la peau de cette composition.

### DESCRIPTION DETAILLEE

A titre préliminaire, on notera que l'expression "compris entre" utilisée tout au long de cette description inclut les valeurs correspondant aux bornes inférieure et supérieure de l'intervalle concerné.

La composition selon l'invention comprend trois phases distinctes, à savoir une phase inférieure aqueuse, une phase intermédiaire huileuse non siliconée et une phase supérieure huileuse non siliconée, dont les constituants seront à présent détaillés.

### Phase inférieure aqueuse

La phase inférieure aqueuse de la composition selon l'invention représente au moins 50% du poids total de la composition, par exemple de 50 à 90%, de préférence de 60 à 85%, plus préférentiellement de 70 à 80%, du poids total de la composition. Elle renferme en outre de 48 à 73% en poids d'eau, de préférence de 50 à 65% en poids d'eau et plus préférentiellement de 50 à 60% en poids d'eau, par rapport au poids total de la composition. En outre, la phase inférieure aqueuse comprend de 2 à 15% en poids de glycérine, par rapport au poids total de la composition. Avantageusement, la glycérine représente de 4 à 10% en poids, par rapport au poids total de la composition. Il a en effet été observé que la glycérine, utilisée dans ces proportions, améliorait la netteté de l'interface entre la phase inférieure aqueuse et la phase intermédiaire huileuse.

Outre les constituants précités, la phase inférieure aqueuse peut comprendre un ou plusieurs constituants additionnels choisis parmi : au moins un tensioactif, qui peut être choisi parmi les tensioactifs non ioniques, avantageusement parmi les alkylpolyglucosides, et/ou parmi les tensioactifs amphotères, tels que la cocamidopropylbétaïne ; au moins un colorant ; au moins un actif, tel qu'un extrait aqueux de plante ; et leurs mélanges.

La phase inférieure aqueuse renferme généralement au moins un ajusteur de pH adapté à amener le pH de la composition dans la plage de 5 à 6,0. Elle peut en outre contenir une faible quantité d'alcool, par exemple de 1 à 10% en poids et de préférence jusqu'à 5% en poids d'éthanol, et/ou un sel, par exemple de 0,1 à 5% en poids de NaCl et/ou de bicarbonate de sodium et de préférence jusqu'à 2% en poids de NaCl et/ou de bicarbonate de sodium, par rapport au poids total de la composition.

En outre, selon une forme d'exécution de l'invention, la phase inférieure aqueuse renferme au moins un colorant adapté à lui conférer une couleur distincte de la phase intermédiaire huileuse.

### Phase intermédiaire huileuse non siliconée

La phase intermédiaire huileuse de la composition selon l'invention renferme de l'huile de ricin, en une quantité pouvant par exemple représenter de 5 à 25% en poids, de préférence de 10 à 20% en poids, plus préférentiellement de 10 à 15% en poids, par rapport au poids total de la composition. L'huile de ricin est essentiellement constituée d'un triglycéride d'acide ricinoléique, c'est-à-dire d'un triglycéride d'acide gras hydroxylé.

Dans une forme d'exécution préférée de l'invention, la phase intermédiaire huileuse renferme en outre au moins une autre huile végétale, dans un rapport en poids de l'huile de ricin à l'huile végétale d'au moins 65:35. Il a en effet été observé qu'une huile végétale, utilisée dans les proportions précitées, permettait d'améliorer le toucher de la composition sans nuire à l'obtention d'une composition triphasique. Le rapport en poids de l'huile de ricin à l'huile végétale est avantageusement d'au moins 70:30, de préférence compris entre 70:30 et 90:10.

Dans le contexte de cette description, on entend par "huile" un composé liquide à température ambiante (25°C) et pression atmosphérique (10⁵ Pa) qui, lorsqu'il est introduit à raison d'au moins 1% en poids dans l'eau à 25°C, n'est pas du tout soluble dans l'eau, ou soluble à hauteur de moins de 10% en poids, par rapport au poids d'huile introduit dans l'eau.

Les huiles végétales peuvent notamment être obtenues par pressage mécanique, souvent à froid, des fruits, noix ou graines de végétaux. Elles sont composées à environ 95 % de triglycérides, le reste étant des mono- et diglycérides et des composés dits insaponifiables tels que les alcools, caroténoïdes, la chlorophylle, les hydrocarbures, stérols et tocophérols. Les triglycérides peuvent eux-mêmes être simples (triesters de glycérol et de trois molécules du même acide gras) ou mixtes (triesters de glycérol et de deux ou trois acides gras différents), les esters mixtes étant majoritaires. Les acides gras contenus dans les huiles végétales sont saturés et/ou insaturés et leur longueur de chaîne est comprise entre 4 et 22 atomes de carbone et pour l'essentiel entre 12 et 18 atomes de carbone.

Des exemples d'huiles végétales utilisables peuvent être choisies parmi les huiles de germe de blé, de tournesol, d'argan, d'hibiscus, de coriandre, de pépins de raisin, de sésame, de maïs, d'abricot, de karité, d'avocat, d'olive, de soja, l'huile d'amande douce, de palme, de colza, de coton, de noisette, de macadamia, de jojoba, de luzerne, de pavot, de potimarron, de courge, de cassis, d'onagre, de lavande, de bourrache, de millet, d'orge, de quinoa, de seigle, de carthame, de bancoulier, de passiflore, de rosier muscat, d'Echium, de cameline, de camélia et leurs mélanges.

Selon une forme d'exécution de l'invention, la phase intermédiaire huileuse renferme en outre au moins un colorant adapté à lui conférer une couleur distincte de la phase inférieure aqueuse et de la phase supérieure huileuse.

Elle peut en outre renfermer au moins un constituant additionnel choisi parmi : les actifs et/ou filtres UV liposolubles ou lipodispersibles en milieu huileux polaire, les anti-oxydants, les conservateurs, les structurants de phase grasse, les tensioactifs, et leurs mélanges.

En revanche, la phase intermédiaire huileuse de la composition selon l'invention est dépourvue de composé siliconé, tel qu'une huile, un élastomère, une gomme ou une résine de silicone. En particulier, elle est dépourvue d'huiles à base d'organopolysiloxanes linéaires, ramifiés ou cycliques. Plus particulièrement, on préfère que la phase intermédiaire huileuse ne comprenne pas d'autre huile qu'une huile de ricin et éventuellement au moins une autre huile végétale.

La phase intermédiaire peut représenter de 8 à 30% en poids, de préférence de 10 à 20% en poids, mieux, de 12 à 20% en poids, par rapport au poids total de la composition.

### Phase supérieure huileuse non siliconée

La phase supérieure huileuse de la composition selon l'invention renferme au moins un alcane linéaire ou ramifié en C₁₀-C₂₀. Ce composé représente avantageusement de 5 à 25% en poids, de préférence de 8 à 20% en poids, plus préférentiellement de 8 à 15% en poids, par rapport au poids total de la composition.

Des exemples d'alcanes linéaires ou ramifiés en C₁₀-C₂₀ peuvent être choisis parmi le décane, le undécane, le dodécane, l'isododécane, le tridécane, le tétradécane, le pentadécane, l'hexadécane, l'isohexadécane, l'heptadécane, l'octadécane, le nonadécane, l'eicosane et leurs mélanges.

Certains de ces alcanes sont volatils, notamment le décane, le undécane et l'isododécane, et d'autres ne sont pas volatils, notamment le tétradécane et le pentadécane. Par "alcane volatil", on entend un alcane ayant une pression de vapeur de l'ordre de 0,001 à 300 mm Hg et de préférence de 0,01 à 10 mm Hg à température ambiante (20°C) et pression atmosphérique. Avantageusement, ces alcanes présentent en outre un point éclair (mesuré selon la norme ASTM D93) inférieur à 100°C, de préférence compris entre 50 et 95°C, par exemple entre 70 et 90°C, voire entre 80 et 90°C et/ou une viscosité cinématique inférieure à 5 cSt, voire comprise entre 1 et 3 cSt, à 40°C. Des exemples d'alcanes linéaires sont les alcanes linéaires en C₁₀ et C₁₃ tels que décrits notamment dans la demande EP 1798213 et commercialisés par la société BASF sous la dénomination commerciale Cetiol^{®} Ultimate ; le mélange d'alcanes linéaires en C₁₂ et C₁₄ commercialisé par la société BIOSYNTHIS sous la dénomination commerciale Vegelight^{®} ; le mélange d'alcanes linéaires en C₁₅-C₁₉ commercialisé par la société SEPPIC sous la dénomination commerciale Emogreen^{®} L15 ; les alcanes linéaires disponibles auprès de la société SASOL sous la dénomination commerciale Parafol^{®} ; et le mélange d'alcanes linéaires en C₁₃-C₁₅ disponible auprès de la société AMYRIS sous la dénomination commerciale Neossance Hemisqualane^{®}.

On préfère selon l'invention utiliser des alcanes linéaires en C₁₀-C₂₀ et plus préférentiellement un mélange d'alcanes linéaires.

En plus de l'huile précitée, qui est par nature apolaire, la composition selon l'invention peut renfermer au moins une autre huile apolaire ou peu polaire. Par "huile apolaire ou peu polaire", on entend au sens de cette description une huile ayant un indice de polarité, c'est-à-dire une tension interfaciale entre cette huile et l'eau, qui est supérieur à 20 mN/m.

Des exemples d'huiles apolaires ou peu polaires sont les dialkyléthers tels que le dicaprylyl éther ; les dialkylcarbonates tels que le dicaprylyl carbonate ; les hydrocarbures tels que le squalane et le squalène ; les polyoléfines telles que le polyisobutène hydrogéné et le polydécène ; les huiles minérales ; les esters d'acides en C₁₂-C₄₄ tels que le laurate d'hexyle ; et leurs mélanges. On préfère toutefois que la phase supérieure huileuse ne renferme pas d'autre huile que le ou les alcane(s) linéaires précités(s).

La phase supérieure huileuse peut représenter de 8 à 30% en poids, de préférence de 10 à 20% en poids, par rapport au poids total de la composition. Il est bien entendu que la sommes des pourcentages des trois phases constituant la composition selon l'invention sera toujours égale à 100%.

Selon un mode de réalisation de l'invention, la phase supérieure huileuse renferme au moins un constituant additionnel choisi parmi : les parfums, les agents anti-oxydants tels que le tocophérol et ses esters, les structurants de phase grasse, les tensioactifs, les actifs et/ou filtres UV liposolubles ou lipodispersibles en milieu huileux apolaire, les conservateurs, et leurs mélanges.

En particulier, selon une forme d'exécution de l'invention, la phase supérieure huileuse renferme au moins un colorant adapté à lui conférer une couleur distincte de la phase intermédiaire huileuse.

Toutefois, tout comme la phase intermédiaire, la phase supérieure de la composition selon l'invention ne renferme pas de composé siliconé. Par ailleurs, on préfère que la phase supérieure huileuse ne contienne pas d'autre constituant que les alcanes linéaires ou ramifiés précités et éventuellement un ou plusieurs parfums, colorants, anti-oxydants et/ou conservateurs.

Comme indiqué précédemment, l'une au moins des trois phases de la composition selon l'invention peut renfermer au moins un actif. On préfère qu'il s'agisse d'un actif anti-âge, en particulier d'un actif adapté à prévenir et/ou traiter les rides, le relâchement cutané et/ou la formation de taches pigmentaires, qui peut notamment être choisi parmi les agents anti-radicalaires, les agents stimulant la différenciation et/ou la prolifération des kératinocytes et/ou des fibroblastes ; les agents stimulant la synthèse de glycosaminoglycanes et/ou de collagène et/ou de fibrilles d'ancrage dermo-épidermique et/ou des fibres élastiques ; les agents prévenant la dégradation du collagène et/ou des glycosaminoglycanes et/ou des fibrilles d'ancrage dermo-épidermique et/ou des fibres élastiques ; les agents anti-glycation ; les agents dépigmentants et/ou inhibant la mélanogénèse ; et leurs mélanges.

Des exemples de tels actifs anti-âge sont notamment : l'acide ascorbique, ses sels, ses éthers et ses esters, notamment le glucoside d'ascorbyle ; l'adénosine ; le ribose ; les extraits de miel; les protéines et glycoprotéines, extraites notamment d'amande douce ; les protéines végétales hydrolysées, notamment issues du riz, des graines d'hibiscus ou du lupin ; les polypeptides et les pseudodipeptides, tels que le chlorhydrate de carcinine, le palmitoyl pentapeptide-4 (Pal-Lys-Thr-Thr-Lys-Ser) et le palmitoyl tripeptide-38 commercialisés notamment par SEDERMA sous les dénominations commerciale Matrixyl^{®} 3000 et Matrixyl^{®} Synthe'6, respectivement, le palmitoyl tripeptide-8 commercialisé par la société LUCAS MEYER sous la référence commerciale Nutrazen^{®}, le pentapeptide-18 commercialisé par la société LIPOTEC sous la dénomination commerciale Leuphasyl^{®} Solution, le sh-decapeptide-9 commercialisé par la société SANDREAM sous la dénomination commerciale Neoendorphin^{®} et le palmitoyl hexapeptide-52 commercialisé par la société INFINITEC sous la référence commerciale X50 Myocept^{®} Powder ; les silanes tels que le mannuronate de méthylsilanol ; les arabinoxylanes, extraits en particulier de farine de seigle et les galactoarabinanes, issus notamment du mélèze ; l'acide hyaluronique et ses sels ; les polyphénols, extraits en particulier de mimosa ; les alpha-hydroxyacides, dont ceux extraits de citron ; les extraits (généralement aqueux) de plantes telles que la marjolaine (*Origanum majorana*), l'immortelle (*Helichrysum italicum*), le trèfle d'eau, la pensée sauvage, la prêle des champs, la Mafane (*Acmella oleracea*), le chardon aux ânes (*Onopordum acanthium*), le millefeuille (*Achillea millefolium,* contenu notamment dans le produit Neurobiox^{®} de la société BASF), l'embelia (*Embelia concinna,* telle que commercialisée par la société SEPPIC), le figuier de Barbarie (*Opuntia ficus indica*, commercialisé notamment par MIBELLE AG BIOCHEMISTRY sous la dénomination commerciale AquaCacteen^{®}), la sauge (*Salvia officinalis,* vendue notamment par PROVITAL GROUP), *Vitex negundo* (commercialisé notamment par les LABORATOIRES EXPANSCIENCE sous la référence commerciale Neurovity^{®}), la châtaigne, la papaye, l'arganier, l'avoine, le tournesol, la pâquerette, la pivoine ou l'aneth ; les extraits aqueux d'algues et notamment de coralline, de janie rouge, d'*Ungaria pinnatifada*, d'*Alaria esculenta* ou de *Nannochlorosis oculata* ; les huiles essentielles, notamment de myrte ou d'immortelle (*Helichrysum italicum*) ; les gluconates de zinc et/ou de cuivre ; et leurs mélanges.

En variante ou en plus, l'actif incorporé dans l'une au moins des phases de la composition selon l'invention peut être choisi parmi les actifs hydratants, tels que l'acide hyaluronique ; l'urée ; les polyols, dont le xylitol, le sorbitol, le propylène glycol ou le mélange de xylityl glucoside, xylitol et anhydroxylitol commercialisé par SEPPIC sous la dénomination commerciale Aquaxyl^{®} ; l'eau de Réotier ; et leurs mélanges.

Les phases aqueuse et huileuses décrites précédemment peuvent être mélangées de manière classique pour l'homme du métier, après homogénéisation de leurs constituants respectifs, pour obtenir la composition selon l'invention. Une fois la composition constituée et placée dans un conditionnement tel qu'un flacon, elle déphase rapidement, après quelques secondes. Après agitation, la composition comporte visuellement une seule phase et, une fois placée au repos, elle déphase à nouveau rapidement.

La composition décrite précédemment peut être utilisée pour le soin, le nettoyage et/ou le parfumage de la peau (du visage et/ou du corps) et/ou des cheveux et/ou le démaquillage de la peau, notamment du visage, y compris éventuellement le contour des yeux. Elle peut par exemple être appliquée sur la peau une à trois fois par jour, par exemple matin et/ou soir. Elle peut avantageusement être conditionnée dans un flacon pourvu d'un pulvérisateur. Il peut s'agir d'une composition rincée ou non rincée.

### EXEMPLES

L'invention sera mieux comprise à la lumière des exemples suivants, qui sont donnés à titre purement illustratif.

### Exemple 1 : Compositions triphasiques

On a mélangé les ingrédients mentionnés ci-dessous dans les proportions pondérales également indiquées ci-dessous, pour préparer différentes compositions selon l'invention.

### Phase aqueuse

| | |
|---|---|
| Eau | 52,0 - 58,5 % |
| Glycérine | 3,0 - 8,0 % |
| Actif anti-âge | qs |
| Sel de mer | 1,0 - 1,2 % |
| Conservateurs | 4,0 - 4,5 % |
| Ajusteur de pH | qs |
| (Total phase aqueuse 64,8 - 73,3 %) | |

### Phase intermédiaire huileuse

| | |
|---|---|
| Huile de ricin | 10,7 - 12,0 % |
| Huile végétale | 3,8 - 5,0 % |
| Actifs anti-âge | qs |
| Anti-oxydant | qs |
| Colorant | qs |
| Parfum | qs |
| (Ratio ricin / huile végétale : 68:32 à 74:26) | |
| (Total phase huileuse 1 : 15,0 -17,7 %) | |

### Phase supérieure huileuse

| | |
|---|---|
| Alcanes linéaires en C₁₅-C₁₉ | 9,0 - 17,50 % |

Les différentes compositions ci-dessus se présentent sous la forme de compositions triphasiques au repos, dans lesquelles les interfaces sont bien nettes et chacune des phases est translucide ou transparente.

### Exemple 2 : Essais comparatifs

On a préparé plusieurs compositions similaires à celles de l'Exemple 1 en faisant varier la nature et/ou la proportion de leurs constituants. L'aspect des compositions obtenues a été évalué visuellement. Les résultats de ces essais sont rassemblés dans le Tableau 1 ci-après.

**Tableau 1**

| Composition | Paramètre modifié | Aspect de la composition au repos |
|---|---|---|
| A | Ratio huile de ricin / huile végétale = 60:40 | Biphase |
| 1A | Ratio huile de ricin / huile végétale = 80:20 et 90:10 | Triphase, interfaces nettes. |
| B | Substitution des alcanes linéaires par le dicaprylyl éther (polarité et densité similaires) | Biphase |
| C | Substitution de l'huile de ricin par une huile d'olive (polarité et densité similaires) | Biphase |
| D | Suppression de la glycérine | Triphase - Phase intermédiaire huileuse opalescente après agitation - Interfaces pas nettes. |

Comme il ressort de ces essais, en particulier de la comparaison des résultats obtenus pour la composition comparative A, d'une part, et les compositions 1A et de l'Exemple 1 (selon l'invention), d'autre part, une huile végétale peut être ajoutée à la composition selon l'invention, sous réserve de respecter un ratio pondéral minimal de 65:35 entre l'huile de ricin et l'huile végétale, faute de quoi il n'est pas possible d'obtenir une composition triphasique. En outre, l'huile de ricin et les alcanes linéaires, respectivement présents dans la phase intermédiaire huileuse et la phase supérieure huileuse, ne peuvent pas être entièrement substitués par une huile de polarité et de densité équivalente sans affecter l'aspect physique de la composition. L'obtention d'une composition triphasique n'est donc pas uniquement liée aux densités et polarités respectives des différentes phases, comme le démontre la comparaison des résultats obtenus pour les compositions comparatives B et C, d'une part, et la composition de l'Exemple 1 (selon l'invention), d'autre part. Enfin, la comparaison de la composition comparative D avec celles de l'Exemple 1 selon l'invention montre que la glycérine constitue un ingrédient critique pour l'obtention d'interfaces nettes.

## Revendications

1. Composition cosmétique triphasique comprenant : (a) une phase inférieure aqueuse représentant au moins 50% du poids de la composition et renfermant de 2 à 15% en poids de glycérine, par rapport au poids total de la composition, (b) une phase intermédiaire huileuse non siliconée renfermant de l'huile de ricin et éventuellement au moins une autre huile végétale, et (c) une phase supérieure huileuse non siliconée renfermant au moins un alcane linéaire ou ramifié en C₁₀-C₂₀, étant entendu que, lorsque la phase intermédiaire huileuse renferme au moins une autre huile végétale, le rapport en poids de l'huile de ricin à l'huile végétale est d'au moins 65:35.

2. Composition selon la revendication 1, **caractérisée en ce que** l'huile de ricin représente de 5 à 25% en poids, de préférence de 10 à 20% en poids, plus préférentiellement de 10 à 15% en poids, par rapport au poids total de la composition.

3. Composition selon la revendication 1 ou 2, **caractérisée en ce que** la glycérine représente de 4 à 10% en poids, par rapport au poids total de la composition.

4. Composition selon l'une quelconque des revendications 1 à 3, **caractérisée en ce que** l'alcane linéaire ou ramifié en C₁₀-C₂₀ représente de 5 à 25% en poids, de préférence de 8 à 20% en poids, plus préférentiellement de 8 à 15% en poids, par rapport au poids total de la composition.

5. Composition selon l'une quelconque des revendications 1 à 4, **caractérisée en ce que** l'alcane linéaire ou ramifié en C₁₀-C₂₀ est choisi parmi le décane, le undécane, le dodécane, l'isododécane, le tridécane, le tétradécane, le pentadécane, l'hexadécane, l'isohexadécane, l'heptadécane, l'octadécane, le nonadécane, l'eicosane et leurs mélanges.

6. Composition selon l'une quelconque des revendications 1 à 5, **caractérisée en ce que** la phase intermédiaire huileuse renferme au moins une huile végétale, dans un rapport en poids de l'huile de ricin à l'huile végétale d'au moins 65:35, de préférence d'au moins 70:30, de préférence encore compris entre 70:30 et 90:10.

7. Composition selon l'une quelconque des revendications 1 à 6, **caractérisée en ce que** l'huile végétale est choisie parmi les huiles de germe de blé, de tournesol, d'argan, d'hibiscus, de coriandre, de pépins de raisin, de sésame, de maïs, d'abricot, de karité, d'avocat, d'olive, de soja, l'huile d'amande douce, de palme, de colza, de coton, de noisette, de macadamia, de jojoba, de luzerne, de pavot, de potimarron, de courge, de cassis, d'onagre, de lavande, de bourrache, de millet, d'orge, de quinoa, de seigle, de carthame, de bancoulier, de passiflore, de rosier muscat, d'Echium, de cameline, de camélia et leurs mélanges.

8. Composition selon l'une quelconque des revendications 1 à 7, **caractérisée en ce que** la phase inférieure aqueuse représente de 50 à 90%, de préférence de 60 à 85% et plus préférentiellement de 70 à 80% du poids total de la composition.

9. Utilisation cosmétique de la composition selon l'une quelconque des revendications 1 à 8 pour le soin, le nettoyage et/ou le parfumage de la peau et/ou des cheveux et/ou le démaquillage de la peau, notamment du visage.

10. Procédé cosmétique de soin, de nettoyage et/ou de parfumage de la peau et/ou des cheveux et/ou de démaquillage de la peau, notamment du visage, comprenant l'application topique sur la peau d'une composition selon l'une quelconque des revendications 1 à 8.

## Patentansprüche

1. Dreiphasige kosmetische Zusammensetzung, die Folgendes umfasst: (a) eine wässrige untere Phase, die mindestens 50 Gew.-% der Zusammensetzung darstellt und von 2 bis 15 Gew.-% Glycerin enthält, bezogen auf das Gesamtgewicht der Zusammensetzung, (b) eine ölige Nicht-Silicon-Zwischenphase, die Ricinusöl und gegebenenfalls mindestens ein anderes pflanzliches Öl enthält, und (c) eine ölige obere Nicht-Silicon-Phase, die mindestens ein lineares oder verzweigtes C₁₀-C₂₀-Alkan enthält, wobei als vereinbart gilt, dass, wenn die ölige Zwischenphase mindestens ein anderes pflanzliches Öl enthält, das Gewichtsverhältnis zwischen Ricinusöl und dem pflanzlichen Öl mindestens 65:35 beträgt.

2. Zusammensetzung nach Anspruch 1, **dadurch gekennzeichnet, dass** das Ricinusöl von 5 bis 25 Gew.-%, vorzugsweise von 10 bis 20 Gew.-%, stärker bevorzugt von 10 bis 15 Gew.-%, bezogen auf das Gesamtgewicht der Zusammensetzung, darstellt.

3. Zusammensetzung nach Anspruch 1 oder 2, **dadurch gekennzeichnet, dass** das Glycerin 4 bis 10 Gew.-%, bezogen auf das Gesamtgewicht der Zusammensetzung, darstellt.

4. Zusammensetzung nach einem der Ansprüche 1 bis 3, **dadurch gekennzeichnet, dass** das lineare oder verzweigte C₁₀-C₂₀-Alkan von 5 bis 25 Gew.-%, vorzugsweise von 8 bis 20 Gew.-%, stärker bevorzugt von 8 bis 15 Gew.-%, bezogen auf das Gesamtgewicht der Zusammensetzung, darstellt.

5. Zusammensetzung nach einem der Ansprüche 1 bis 4, **dadurch gekennzeichnet, dass** das lineare oder verzweigte C₁₀-C₂₀-Alkan aus Decan, Undecan, Dodecan, Isododecan, Tridecan, Tetradecan, Pentadecan, Hexadecan, Isohexadecan, Heptadecan, Octadecan, Nonadecan, Eicosan und deren Mischungen ausgewählt ist.

6. Zusammensetzung nach einem der Ansprüche 1 bis 5, **dadurch gekennzeichnet, dass** die ölige Zwischenphase mindestens ein pflanzliches Öl in einem Gewichtsverhältnis zwischen Ricinusöl und dem pflanzlichen Öl von mindestens 65:35, vorzugsweise mindestens 70:30, noch mehr bevorzugt zwischen 70:30 und 90:10, enthält.

7. Zusammensetzung nach einem der Ansprüche 1 bis 6, **dadurch gekennzeichnet, dass** das pflanzliche Öl aus Weizenkeim-, Sonnenblumen-, Argan-, Hibiskus-, Koriander-, Traubenkern-, Sesam-, Mais-, Aprikosen-, Karite-, Avocado-, Oliven-, Sojalöl, Süßmandel-, Palm-, Raps-, Baumwollsamen-, Haselnuss-, Macademia-, Jojoba-, Luzerne-, Mohn-, Hokkaidokürbis-, Kürbiskernöl, Öl der schwarzen Johannisbeere, Nachtkerzen-, Lavendel-, Borretsch-, Goldhirse-, Gersten-, Quinoa-, Roggen-, Distel-, Kukuinuss-, Passionsblumen-, Hagebutten-, Echium-, Leindotter-, Kamelienöl und deren Mischungen ausgewählt ist.

8. Zusammensetzung nach einem der Ansprüche 1 bis 7, **dadurch gekennzeichnet, dass** die wässrige untere Phase von 50 bis 90 Gew.-%, vorzugsweise von 60 bis 85 Gew.-% und stärker bevorzugt von 70 bis 80 Gew.-% des Gesamtgewichts der Zusammensetzung darstellt.

9. Kosmetische Verwendung der Zusammensetzung nach einem der Ansprüche 1 bis 8 zur Pflege, Reinigung und/oder Parfümierung der Haut und/oder der Haare und/oder zur Make-up-Entfernung von der Haut, insbesondere vom Gesicht.

10. Kosmetisches Verfahren zur Pflege, Reinigung und/oder Parfümierung der Haut und/oder der Haare und/oder zur Make-up-Entfernung von der Haut, insbesondere vom Gesicht, welches das topische Auftragen einer Zusammensetzung nach einem der Ansprüche 1 bis 8 auf die Haut umfasst.

## Claims

1. A three-phase cosmetic composition comprising: (a) an aqueous lower phase representing at least 50% of the weight of the composition and containing from 2% to 15% by weight of glycerol, relative to the total weight of the composition, (b) a non-silicone oily intermediate phase containing castor oil and optionally at least another plant oil, and (c) a non-silicone oily upper phase containing at least one linear or branched C₁₀-C₂₀ alkane, it being understood that when the oily intermediate phase contains at least another plant oil, the weight ratio of the castor oil to the plant oil is at least 65:35.

2. The composition as claimed in claim 1, **characterized in that** the castor oil represents from 5% to 25% by weight, preferably from 10% to 20% by weight, more preferentially from 10% to 15% by weight, relative to the total weight of the composition.

3. The composition as claimed in claim 1 or 2, **characterized in that** the glycerol represents from 4% to 10% by weight, relative to the total weight of the composition.

4. The composition as claimed in any one of claims 1 to 3, **characterized in that** the linear or branched C₁₀-C₂₀ alkane represents from 5% to 25% by weight, preferably from 8% to 20% by weight, more preferentially from 8% to 15% by weight, relative to the total weight of the composition.

5. The composition as claimed in any one of claims 1 to 4, **characterized in that** the linear or branched C₁₀-C₂₀ alkane is chosen from decane, undecane, dodecane, isododecane, tridecane, tetradecane, pentadecane, hexadecane, isohexadecane, heptadecane, octadecane, nonadecane, eicosane and mixtures thereof.

6. The composition as claimed in any one of claims 1 to 5, **characterized in that** the oily intermediate phase contains at least one plant oil, in a weight ratio of the castor oil to the plant oil of at least 65:35, preferably of at least 70:30, more preferably between 70:30 and 90:10.

7. The composition as claimed in any one of claims 1 to 6, **characterized in that** the plant oil is chosen from wheatgerm oil, sunflower oil, argon oil, hibiscus oil, coriander oil, grapeseed oil, sesame oil, corn oil, apricot oil, shea oil, avocado oil, olive oil, soybean oil, sweet almond oil, palm oil, rapeseed oil, cottonseed oil, hazelnut oil, macadamia oil, jojoba oil, alfalfa oil, poppy seed oil, red kuri squash oil, pumpkin oil, blackcurrant oil, evening primrose oil, lavender oil, borage oil, millet oil, barley oil, quinoa oil, rye oil, safflower oil, candlenut oil, passionflower oil, musk rose oil, echium oil, camelina oil, camellia oil and mixtures thereof.

8. The composition as claimed in any one of claims 1 to 7, **characterized in that** the aqueous lower phase represents from 50% to 90%, preferably from 60% to 85% and more preferentially from 70% to 80% of the total weight of the composition.

9. A cosmetic use of the composition as claimed in any one of claims 1 to 8, for caring for, cleansing and/or perfuming the skin and/or hair and/or for removing makeup from the skin, notably the face.

10. A cosmetic method for caring for, cleansing and/or perfuming the skin and/or hair and/or for removing makeup from the skin, notably the face, comprising the topical application of a composition as claimed in any one of claims 1 to 8 on the skin.
